# FASCICULE DE BREVET EUROPEEN

(11) **EP 3 197 899 B1**
(45) Date de publication et mention de la délivrance du brevet: **27.06.2018**
(21) Numéro de dépôt: 15780924.5
(22) Date de dépôt: 22.09.2015
(51) Int. Cl.: C07D 493/04, C08L 27/06, C08K 5/1535

(54) **DIESTERS EPOXYDES DE L'ISOSORBIDE ET LEUR UTILISATION COMME PLASTIFIANT DANS DES COMPOSITIONS A BASE DE PVC**
ISOSORBID EPOXIDDIESTER UND IHRE VERWENDUNG ALS WEICHMACHER IN PVC-ZUSAMMENSETZUNGEN
ISOSORBIDE EPOXIDE DIESTERS, AND THE USE THEREOF AS A PLASTICIZER IN PVC COMPOSITIONS

(30) Priorité: 23.09.2014 FR 1458962
(43) Date de publication de la demande: 02.08.2017
(73) Titulaire: Roquette Frères, 62136 Lestrem (FR)
(72) Inventeur: WYART, Hervé, F-62149 Cuinchy (FR); BUFFE, Clothilde, F-59160 Lomme (FR); BROCARD, Juliette, F-62400 Béthune (FR)
(74) Mandataire: Cabinet Plasseraud
(86) Numéro de dépôt international: PCT/FR2015/052538
(87) Numéro de publication internationale: WO 2016/046490

(56) Documents cités:
- WO-A1-99/45060
- US-A- 3 225 067
- US-A1- 2006 020 062

## Description

L'invention concerne de manière générale des esters époxydés de l'isosorbide, et leur utilisation comme additif dans des formulations de PVC, notamment en tant que plastifiant.

Le polychlorure de vinyle ou chlorure de polyvinyle est un polymère thermoplastique de grande consommation, amorphe ou faiblement cristallin, connu sous l'acronyme anglo-saxon « PVC » pour « PolyVinyl Chloride ». Il est en général formulé avec des additifs comme des stabilisants, des charges, des pigments et des plastifiants, et est aujourd'hui largement répandu dans de très nombreuses applications.

Le PVC est un matériau rigide qui peut être utilisé pour fabriquer des tuyaux, ceux-ci représentant plus de 40 % de la consommation mondiale de PVC. On peut aussi lui ajouter des plastifiants dont le rôle est d'améliorer sa souplesse, mais aussi son allongement à la rupture, sa tenue au froid et aux chocs, et plus généralement sa mise en oeuvre. Il peut alors se présenter sous forme dite « expansée » (souvent appelée Forex) utilisée notamment pour réaliser des enseignes publicitaires, ou sous forme de films plastifiés servant comme adhésifs ou matériaux d'emballage, et enfin sous forme de PVC souple proprement dit, principalement dans des applications de revêtements de sols et de plafonds.

La plupart des plastifiants mis en oeuvre dans les PVC sont des liquides organiques non volatils ou à faible point de fusion, dont le mécanisme de fonctionnement repose sur une réduction des forces intermoléculaires dans la résine PVC, permettant ainsi aux macromolécules de chlorure de vinyle de « glisser » plus librement les unes sur les autres. Par augmentation du « volume libre » entre les chaînes polymériques, ils abaissent la température de transition vitreuse du PVC, ce qui contribue à assouplir ce dernier.

Ces plastifiants sont à base d'esters d'acides polycarboxyliques avec des alcools aliphatiques linéaires ou ramifiés. Les plus connus pour le PVC sont les phtalates, les composés à base d'adipate et de trimellitate. A titre d'exemples de plastifiants à base de phtalate, on pourra citer le phtalate de di-isononyle (DINP), le phtalate di-2-éthylhexyle (DEHP), le phtalate de dibutyle (DBP), le phtalate diisodécylique (DIDP), le butyle phtalate de dibenzyle (BBP).

Les phtalates sont des polluants organiques très répandus dans l'environnement des zones urbaines. L'étiquetage de ces composés nécessite la mention « Toxique » et certains portent aussi la mention « Dangereux pour l'environnement ». L'utilisation de certains phtalates dans les articles de puériculture ou les jouets destinés aux enfants de moins de 3 ans a notamment été interdite depuis quelques années et est fréquemment révisée (voir la directive 2005/84/CE [PDF] dans l'Union Européenne et le décret de transposition n° 2006-1361 du 9 novembre 2006 en France).

Pour les autres matières plastiques, aucune réglementation n'est appliquée car les doses auxquelles sont employés ces plastifiants ne sont pas considérées comme dangereuses. Il n'en reste pas moins que les phtalates les plus répandus (DEHP, DBP, DINP, DIDP et BBP) font toujours l'objet de nombreuses études par divers organismes internationaux (Food and Drug Administration, Bureau européen des substances chimiques et Institut National de Santé Publique au Québec).

Aussi, il existe un besoin constant de trouver de nouveaux plastifiants pour les matériaux thermoplastiques et notamment le PVC, qui présentent des propriétés de plastification au moins équivalentes à celles des phtalates, tout en privilégiant une origine bio-sourcée.

A ce titre, les 1,4 - 3,6 dianhydrohexitols et plus particulièrement les esters de 1,4 - 3,6 dianhydrohexitols, et de manière encore plus préférée les esters d'acides gras du composé particulier qu'est l'isosorbide constituent une piste privilégiée.

En ce qui concerne les 1,4 - 3,6 dianhydrohexitols, ces composés également appelés isohexides sont obtenus par déshydratation interne de sucres hydrogénés en C₆ (hexitols) tels que le sorbitol, le mannitol et l'iditol. Dans la présente demande, le terme « 1,4 - 3,6 dianhydrohexitols » englobe l'isosorbide (1,4 - 3,6-dianhydro-sorbitol), l'isomannide (1,4 - 3,6 dianhydro-mannitol), l'isoidide (1,4 - 3,6-dianhydro-iditol) de formules suivantes, ainsi que les mélanges de ces produits :

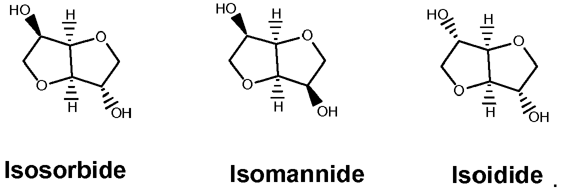

En ce qui concerne les acides gras ou huiles végétales, on rappelle que ces produits désignent des acides carboxyliques à chaîne aliphatique. Les acides gras naturels possèdent une chaîne carbonée de 4 à 36 atomes de carbone et typiquement en nombre pair. Les acides gras sont présents dans les graisses animales et les graisses végétales, les huiles végétales ou les cires, sous forme d'esters.

L'utilisation de dérivés de 1,4 - 3,6 dianhydrohexitols comme plastifiants de polymères a déjà été décrite dans le document WO 99/45060. Les exemples de cette demande décrivent des plastifiants liquides à température ambiante : le dioctanoate d'isosorbide, le dibutanoate d'isosorbide, le dihexanoate d'isosorbide et le di(2-ethylhexanoate) d'isosorbide. Ces plastifiants sont également décrits dans le document WO 2008/095571 A1, qui relate des diesters aliphatiques à 9 atomes de carbone. Le document « Préparation of plasticizers from carbohydrate sources. I. Levulinic acid esters. II. Sorbide esters » (Hachihama et al., Technology reports of the Osaka University, Vol. 3, n°72, 1953, pages 191-200) décrit des esters aliphatiques à 8 atomes de carbone ainsi que des esters aliphatiques à 10 atomes de carbone. Le brevet US 2,387,842 décrit quant à lui des diesters aliphatiques mixtes, ceux-ci étant également utiles comme plastifiants. Des mélanges de ce type d'esters sont également décrits dans les demandes WO 2014/ 080151 A1 et WO 2014/080152 A1, de tels produits pouvant être obtenus à partir de 1,4 - 3,6 dianhydrohexitols et de mélanges d'acides gras.

Aussi, il existe un besoin constant de mettre au point de nouveaux produits à base d'esters de 1,4 - 3,6 dianhydrohexitols susceptibles d'être valorisés comme plastifiants dans des compositions à base de PVC. A cet égard, la société demanderesse est parvenue à synthétiser de telles molécules. Celles-ci consistent en des diesters époxydés d'acides gras de l'isosorbide, plus précisément en des diesters époxydés des acides oléique, linoléique et linolénique de l'isosorbide, ainsi que leurs mélanges, les composés individuels étant ceux donnés par les formules (I), (II) et (III) suivantes :

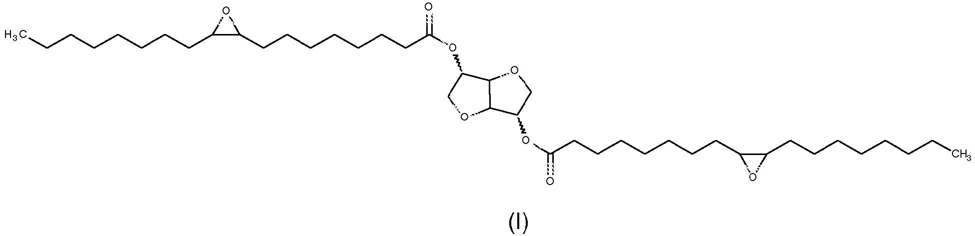

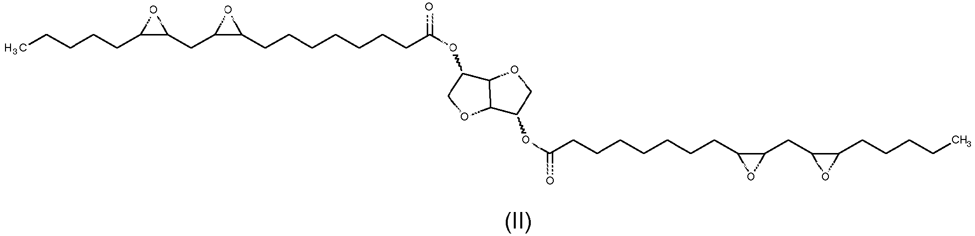

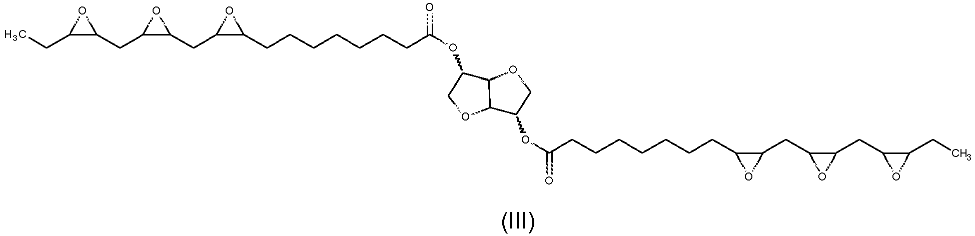

La société demanderesse indique que des esters époxydés d'acides gras de 1,4 - 3,6 dianhydrohexitols sont par ailleurs déjà connus. Ainsi, le document US 3,225,067 décrit-il des diesters époxydés de polyoxyéthylène d'isosorbide contenant de 2 à 8 groupements oxyéthylène par mole et 2 moles d'acides gras insaturé éthylénique avec un degré d'époxydation d'au moins 25 % des groupements insaturés.

On connaît aussi le document WO 2013 / 092655 qui décrit un mélange d'esters de dianhydrohexitol, ledit mélange ayant une longueur moyenne de chaîne comprise entre 8,3 et 9,2, lesdits esters pouvant être par ailleurs époxydés.

On connaît enfin le document US 2006 020062 qui décrit des esters époxydés d'acides gras et d'huiles végétales qui peuvent être dans certains cas des esters époxydés d'isosorbide. Ces produits peuvent être mis en oeuvre en tant que plastifiant dans des compositions à base de PVC.

Ceci étant et au meilleur des connaissances de la demanderesse, aucun de ces documents pas plus qu'un autre de l'art antérieur ne décrit ni ne divulgue un des 3 quelconques composés précités et représentés à travers les formules (I), (II) et (III). En outre et de manière à la fois surprenante et avantageuse, les composés objets de la présente demande s'avèrent être très compatibles avec le PVC et avoir une très bonne aptitude à le plastifier, contrairement à leurs homologues non époxydés (e.g. les esters de 1,4 - 3,6 dianhydrohexitols n'ayant pas subi la réaction d'époxydation selon la présente invention).

Dans le cas de la présente demande, la compatibilité du composé avec le PVC est caractérisée par le point sec (ou dry-blending time en anglais) relevé pendant l'étape de mélange à sec, qui est le temps depuis l'introduction du composé dans la résine PVC jusqu'à son absorption complète par cette dernière. Une faible compatibilité d'un plastifiant avec le PVC peut conduire à une perte de souplesse de la plaque de PVC dans le temps, à l'apparition d'une opacité (cas d'une formule non chargée et non pigmentée) et/ou d'un toucher gras en surface. En outre, un point sec élevé peut, selon le procédé du transformateur de PVC, risquer de ralentir le processus de fabrication du PVC plastifié et ainsi induire une perte de productivité.

L'aptitude du composé à plastifier le PVC est quant à elle caractérisée en ce que la plaque de PVC obtenue après le procédé de transformation est cohésive, souple et résistante à la fois.

Aussi, un premier objet de la présente invention concerne les diesters époxydés de l'isosorbide de formules (I), (II) et (III) précités.

Un autre objet de la présente invention concerne tous les mélanges de ces différents produits, c'est-à-dire le mélange de deux ou trois composés parmi les composés de formule (I), de formule (II) et de formule (III).

Ces mélanges peuvent présenter typiquement un indice d'iode inférieur à 10 g I2 / 100 g, préférentiellement inférieur à 6 g I2 / 100 g et un taux d'oxirane supérieur à 3 %.

L'indice d'iode est mesuré selon la norme NF/EN/ISO 3961 (14 septembre 2013). Il est exprimé en g d'iode pour 100 g de produit.

Le % d'oxirane est défini comme le % en poids d'oxygène par rapport au poids total du produit et est déterminé par RMN.

Un autre objet de la présente invention concerne l'utilisation de ces produits ou de leurs mélanges comme plastifiant dans des compositions à base de PVC.

Les composés de formules (I), (II) et (III) et leurs mélanges sont susceptibles d'être obtenus à partir de procédés connus par ailleurs.

La première étape d'un tel procédé consiste en une réaction d'estérification de l'isosorbide. Cette étape peut être réalisée par toute méthode connue d'estérification de 1 ,4 : 3,6-dianhydrohexitol et notamment de l'isosorbide par un acide carboxylique, ledit procédé étant caractérisé en ce qu'on met en oeuvre en lieu et place de l'acide un des acides oléique, linoléique et linolénique, ou un de leurs mélanges. Ces acides peuvent notamment être les produits commercialisés par la société OLEON sous les noms Nouracid 1880, Nouracid HE 30 et Nouracid LE 80.

Des méthodes d'estérification sont décrites par exemple dans les documents WO 99 / 45060 A1 et WO 2006 / 103338 A1.

On peut réaliser l'étape d'estérification en présence d'au moins un catalyseur acide. Ce dernier peut être de nature très variée : il peut s'agir d'un acide choisi parmi l'acide hypophosphoreux, l'acide chlorhydrique, l'acide sulfurique, l'acide para-toluène sulfonique (APTS), l'acide méthanesulfonique (AMS), l'acide trifluorométhanesulfonique, l'acide trifluoroacétique, l'acide trichloroanétique, l'éthyl-2-hexanoate d'étain, l'acide phosphotungstique et l'acide silicotungstique ou un mélange de ces acides ou une résine macroporeuse ou non macroporeuse comprenant au moins un de ces acides. De préférence, le catalyseur comprend de l'acide hypophosphoreux. Dans le cas de mélanges de catalyseurs, ils peuvent être introduits dans le milieu réactionnel simultanément, ou non.

Le % massique de catalyseur acide peut varier de 0,05 à 20 % par rapport à la masse d'isosorbide introduite dans le réacteur, par exemple de 0,1 à 10 %. La température dans le réacteur peut aller de 90 à 200 °C, généralement de 100 à 150 °C. Pour réaliser la réaction d'estérification, on élimine classiquement l'eau afin de permettre la formation du diester, cette élimination pouvant se faire par exemple à travers la distillation du milieu réactionnel. Afin de faciliter cette élimination, on peut placer le milieu réactionnel sous vide, par exemple sous un vide correspondant à une dépression comprise entre 10 et 200 mbar. On peut faire varier les conditions réactionnelles telles que le niveau de vide et la température lors de la réaction.

La réaction d'estérification est généralement poursuivie jusqu'à obtenir un degré de conversion en diester d'isosorbide d'au moins 90%. Elle peut durer de 1 à 10 heures.

On peut également réaliser une étape de neutralisation du catalyseur, en introduisant une base, par exemple de la soude, dans des quantités molaires au moins équivalentes aux quantités molaires de catalyseur introduit.

Le procédé de fabrication peut comprendre en outre une étape ultérieure de purification de la composition de diester. Celle-ci consiste avantageusement en au moins une étape d'évaporation, par exemple par distillation, permettant d'éliminer la plus grande partie ou la quasi-totalité de l'acide encore présent à l'issue de l'étape d'estérification. Lors de cette étape, la composition de diester peut être soumise une température comprise entre 100 et 250 °C et à une dépression comprise entre 0,1 et 50 mbar. De préférence, cette étape se fait dans un évaporateur continu. Un tel évaporateur, par exemple de type " à flot tombant " ou mieux, de type à film raclé ou " short path ", permet de limiter les températures et temps de séjour auxquels est soumise la composition issue de l'étape d'estérification.

Le procédé peut également comprendre une étape de décoloration de la composition de diester, par exemple en utilisant des charbons actifs ou de l'eau oxygénée. Le traitement par du charbon actif peut être réalisé par mise en contact de la composition avec 1 à 3 % en poids de charbon actif. La température lors de ce traitement peut être voisine de 100 °C. La durée en est généralement comprise entre 10 minutes et 1 heure. A la fin du traitement, le charbon actif est éliminé par filtration. Un traitement classique de décoloration par eau oxygénée consiste par exemple à introduire dans la composition à décolorer, sur une période allant par exemple de 30 à 60 minutes, de 0,5 à 2 % d'eau oxygénée à 100 %, à une température comprise entre 90 et 150 °C, puis on agite la composition pendant 1 à 2 heures à cette température. Quand on souhaite associer ces deux types de traitement de décoloration, le traitement à l'eau oxygénée précède de préférence celui au charbon actif. Ce dernier permet en effet de détruire les peroxydes éventuellement présents.

La deuxième étape du procédé consiste en l'époxydation de la composition de diester d'isosorbide obtenue précédemment. Cette étape peut être réalisée selon tous les procédés connus d'époxydation de diester d'isosorbide. Le document US 2006 020062 déjà cité dans la présente Demande en est une illustration.

La réaction d'époxydation consiste à mettre la composition de diester obtenue précédemment en présence d'au moins un agent contenant la fonction peroxyde. Cet agent peut être notamment choisi parmi le peroxyde d'hydrogène, les acides peroxycarboxyliques ou les alkyles hydroperoxydes. On préfèrera le peroxyde d'hydrogène.

En présence de peroxyde d'hydrogène, on ajoute un acide, notamment l'acide formique ou l'acide acétique afin de former in-situ le peracide correspondant qui est plus réactif.

La réaction peut être catalysée en présence d'un autre acide comme l'acide sulfurique ou une résine cationique forte, notamment la résine commercialisée par la société DOW sous le nom Amberlyst 15.

On pourra choisir d'ajouter des tensio-actifs pour faciliter la dispersion de la phase huile au sein de la phase aqueuse.

L'avancement de la réaction peut être suivi par la disparition du signal RMN entre 2.8 et 3.2 ppm correspondant aux protons éthyléniques.

La réaction se fait entre 20 et 60°C, préférentiellement entre 30 et 50°C à pression atmosphérique.

En fin de réaction, pour faciliter les lavages, la phase organique peut être diluée dans un solvant organique non miscible dans l'eau comme l'acétate d'éthyle. La présence de peroxyde résiduel est éliminée par lavage à l'aide d'une solution de bisulfite de sodium. L'acide acétique ou formique est éliminé par lavage à l'eau.

Le produit est récupéré après évaporation du solvant organique sous pression réduite au rotavapor.

Un autre objet de la présente invention concerne l'utilisation des produits précités ou de leurs mélanges comme plastifiant dans des formulations de PVC.

Un autre objet de la présente invention concerne une formulation de PVC comprenant du polychlorure de vinyle et l'un des composés de formule (I), (II) ou (III) selon l'invention ou l'un de leurs mélanges.

Un autre objet de la présente invention concerne un article plastique, fini ou semi-fini, comprenant du polychlorure de vinyle et l'un des composés de formule (I), (II) ou (III) selon l'invention ou l'un de leurs mélanges.

Par « formulation de PVC » on entend dans la présente demande des formulations thermoplastiques dont le composant polymère majoritaire est le PVC (typiquement une composition polymérique contenant au moins 80 % en poids de PVC).

Outre les composés selon la présente invention, ladite formulation de PVC peuvent contenir d'autres additifs classiques tels que des charges minérales ou organiques stabilisants, pigments, ignifugeants ou lubrifiants. Elle peut se présenter sous forme de mélange sec (dry blend en anglais), de granulés ou de plastisols.

Les composés objets de la présente invention ayant été synthétisés, ils peuvent ensuite être mis en oeuvre dans des formulations de PVC. Ils sont classiquement mélangés au PVC selon différents procédés. Le PVC peut ensuite être transformé en objet par différentes techniques de transformation des matériaux thermoplastiques, et en particulier par extrusion, par calandrage ou encore par enduction via un procédé de type plastisol.

Afin d'obtenir ce mélange thermoplastique, on mélange le PVC avec le plastifiant en apportant à ce système de l'énergie, sous forme de température et d'énergie mécanique. Dans le cas de l'extrusion, ce mélange se fait dans un système fermé. Dans le cas d'un mélange sur cylindres, ce mélange se fait dans un système ouvert. Le polymère peut être ensuite mis en forme, par exemple par des procédés de thermoformage ou de calandrage. Généralement, on réalise une étape de mélange à sec (en anglais dry blend) avant l'étape de mélange thermomécanique. Selon le procédé plastisol, on réalise généralement un mélange pour former une pâte de PVC, puis cette pâte est mise en forme par une étape d'enduction ou de moulage, et la pâte est ensuite chauffée dans un four pour former la pièce.

Les exemples qui suivent permettront de mieux appréhender la présente invention, sans pour autant en limiter la portée.

### EXEMPLES

### Méthodes analytiques

Dans tous les exemples qui suivent, les méthodes analytiques utilisées sont les suivantes.

La quantité massique en diester obtenue après la réaction d'estérification est mesurée par chromatographie phase gazeuse. La colonne utilisée est une ZB1HT de longueur 30 mètres, de diamètre interne 0.32 mm, d'épaisseur de film 0.25 µm. La quantité massique en diester est donnée, par le ratio de la somme des aires des composés correspondant aux diesters d'isosorbide sur la somme des aires de la totalité des composés.

Le degré d'insaturation de la chaine grasse du diester d'isosorbide est déterminé par la mesure de l'indice d'Iode selon la norme NF/EN/ISO 3961 (14 septembre 2013). Il est exprimé en g d'iode pour 100 g de produit.

Le % d'oxirane est défini comme le % en poids d'oxygène par rapport au poids total du produit et est déterminé par RMN.

### Exemple 1

Cet exemple concerne la synthèse de diesters d'isosorbide à partir d'isosorbide et de différents acides gras commerciaux qui sont des coupes en acides oléique, linoléique et linolénique.

### Essai n° 1

Dans un réacteur en verre de 1 litre muni d'une double-enveloppe alimentée par un bain thermostaté à circulation d'huile, d'une pâle d'agitation, d'un thermomètre, d'une tête de distillation associée à un réfrigérant et à une recette de distillation, on introduit 146 g d'isosorbide (1 mole) et 564g (2 moles) de Nouracid 1880 (acide gras riche en acide oléique), fourni par la société OLEON, contenant 79.3 % en poids d'acide oléique (C18 :1), 12.1 % d'acide linoléique (C18 :2) et 0.1 % d'acide linolénique (C18 :3).

Le système d'agitation est mis en fonctionnement à 400 tr/mn, ainsi que le bain thermostaté avec une consigne de 100°C. Quand la température du milieu réactionnel atteint 60°C, on ajoute 2,92 g d'acide p-toluène sulfonique (APTS) monohydrate (2 % commercial par rapport à l'isosorbide sec) et 0,90 g d'acide hypophosphoreux à 50 % soit 0,3 % en sec par rapport à l'isosorbide sec. La consigne du bain thermostaté est ensuite fixée à 160°C et l'agitation à 650 tr/mn. L'ensemble du montage est alors relié à une pompe à vide munie d'un vacuomètre dont on fixe la consigne à 100 mbar.

Lorsque la température du milieu réactionnel atteint 115°C environ, l'eau issue de la réaction d'estérification est distillée et recueillie dans la recette. Le vide est alors progressivement abaissé sur 4 heures jusqu'à 30 mbar et la température de consigne du bain thermostaté augmentée de 10°C par heure pour atteindre 200°C. Après 4 heures, la température du milieu réactionnel est de 185°C. On fixe alors la température de consigne du bain thermostaté à 220°C et on poursuit la réaction pendant 2 heures. Après 6 heures de réaction, la température du milieu réactionnel est de 210°C. Le milieu réactionnel est ensuite refroidi jusqu'à 100°C environ, et on neutralise les acidités fortes de l'APTS et de l'acide hypophosphoreux par l'ajout de 1,8 g de soude à 50 %.

On distille ensuite sous vide l'acide gras qui n'a pas réagi (Pression <2 mbar) avec une température de double-enveloppe de 250°C. Après refroidissement à 100°C, le produit est décoloré par un traitement au charbon actif. La composition ainsi purifiée, présente une quantité massique en diester d'isosorbide de 99.0 % et un indice d'iode de 79 g I2 pour 100 g de produit.

### Essai n° 2

On réalise cet essai selon le protocole opératoire de l'essai précédent en substituant l'acide gras riche en acide oléique par du Nouracid HE30 (OLEON), acide gras riche en acide linoléique, contenant 29.2% en poids d'acide oléique (C18 :1), 58.3% d'acide linoléique (C18 :2) et 0.4% d'acide linolénique (C18 :3).

La composition finale obtenue présente une quantité massique en diesters d'isosorbide de 97.7 % et un indice d'iode de 110.9 g I2 pour 100 g de produit

### Essai n° 3

On réalise un essai 3 selon le protocole opératoire de l'essai n° 1 en substituant l'acide gras riche en acide oléique par du Nouracid LE80 (OLEON), acide gras riche en acide linolénique, contenant 20.3% en poids d'acide oléique (C18 :1), 18% d'acide linoléique (C18 :2) et 50.1% d'acide linolénique (C18 :3).

La composition finale obtenue présente une quantité massique en diesters d'isosorbide de 97.2 % et un indice d'iode de 151 g I2 pour 100 g de produit.

### Exemple 2

Cet exemple concerne la réaction d'époxydation des esters obtenus au cours de l'exemple précédent. On obtient ici les produits selon la présente invention.

### Essai n° 4

Dans un réacteur en verre de 1 litre muni d'une double-enveloppe alimenté par un bain thermostaté à circulation d'eau, d'une pâle d'agitation, d'un thermomètre et d'un réfrigérant, on introduit 100 g du composé obtenu à l'essai n° 1 (0.31 mole d'insaturations), 352.5 g d'une solution aqueuse d'eau oxygénée à 30 % (3.1 moles), 35.8 g d'acide formique (0.78 mole) et 0.2 g de Tween 20 (0.2 % massique par rapport au diester).

Le système d'agitation est mis en fonctionnement à 200 tr/mn, ainsi que le bain thermostaté avec une consigne de 30°C. Après 24 h de réaction, le milieu réactionnel est extrait à l'acétate d'éthyle. La phase organique est lavée à l'aide d'une solution aqueuse de bisulfite de sodium puis avec de l'eau. La phase organique est séchée avec du sulfate de magnésium anhydride, filtrée et concentrée au rotavapor.

Le produit fini possède un indice d'iode de 1.6 g I2/100 g de brut. L'analyse RMN confirme la présence de motif époxydes (signal entre 2.8 et 3.2 ppm). Le taux d'oxirane est de 3 %.

### Essai n° 5

Dans un réacteur en verre de 1 litre muni d'une double-enveloppe alimenté par un bain thermostaté à circulation d'eau, d'une pâle d'agitation, d'un thermomètre et d'un réfrigérant, on introduit 200 g du composé obtenu à l'essai n° 2 (0.87 mole d'insaturations), 148.6 g d'une solution aqueuse d'eau oxygénée à 30 % (1.3 moles), 20.1 g d'acide formique (0.44 mole) et 20 g d'Amberlyst 15 (10 % massique par rapport au diester).

Le système d'agitation est mis en fonctionnement à 200 tr/mn, ainsi que le bain thermostaté avec une consigne de 30 °C. Après 24 h de réaction, le milieu réactionnel est extrait à l'acétate d'éthyle. La phase organique est lavée à l'aide d'une solution aqueuse de bisulfite de sodium puis avec de l'eau. La phase organique est séchée avec du sulfate de magnésium anhydride, filtrée et concentrée au rotavapor.

Le produit fini possède un indice d'iode de 0.6 g I2/100 g de brut. L'analyse RMN confirme la présence de motif époxydes (signal entre 2.8 et 3.2 ppm). Le taux d'oxirane est de 3.4 %.

### Essai n° 6

On réalise cet essai selon le protocole opératoire de l'essai précédent mais à partir du produit obtenu suivant l'essai n° 3.

Le produit fini possède un indice d'iode de 3.6g I2/100 g de brut. L'analyse RMN confirme la présence de motif époxydes (signal entre 2.8 et 3.2 ppm). Le taux d'oxirane est de 5.2%.

### Exemple 3

Cet exemple décrit la mise en oeuvre comme plastifiant, dans une formulation de PVC :
- des 3 esters d'isosorbide non époxydés obtenus selon les essais n° 1 à 3
- des 3 esters époxydés d'isosorbide obtenus selon les essais n° 4 à 6

La formulation de PVC plastifié selon l'invention est composée des produits suivants :
PVC MARVYLAN® S7102 : 100 parts
Stabilisant BAEROSTAB® NT 319P (Ca/Zn poudre) : 1,5 part
Co-stabilisant LANKROFLEX® E 2307 (huile de soja époxydée) : 2 parts
Plastifiant : 34 parts

La préparation des plaques de PVC plastifié se fait en plusieurs étapes.
1) Préparation d'un mélange sec (dry blend en anglais) de PVC plastifié :
   Une masse de 500 g de PVC (poudre) est introduite dans un mélangeur planétaire de type PLANETMIX 500 (Sté Thermo Scientific) équipé d'un circuit de régulation de la température, avec la quantité correspondante de stabilisant thermique et de co-stabilisant thermique. Lorsque la température du mélange atteint 85°C, le plastifiant est versé sur toute la surface de la poudre de PVC. La préparation est ensuite mélangée pendant encore 8 min après l'absorption totale du plastifiant dans le PVC.
   Lors de cette étape de mélange à sec, la mesure du point sec caractérise la vitesse d'absorption du plastifiant dans le PVC et s'obtient comme suit. Le mélangeur PLANETMIX utilisé permet de suivre l'évolution du couple de son moteur pendant toute l'étape de mélange. Dès l'instant où le plastifiant est introduit dans le PVC, le couple augmente au fur et à mesure que le plastifiant est ajouté, jusqu'à atteindre un maximum quand l'intégralité du plastifiant a été versée. Le couple commence ensuite à diminuer au fur et à mesure que le plastifiant est absorbé par le PVC. Quand il a été intégralement absorbé, le couple mesuré atteint un minimum. Le point sec est alors défini par le temps mis par le mélange, depuis l'introduction du plastifiant, pour attendre ce couple minimum, et caractérise ainsi la vitesse d'absorption du plastifiant, liée à sa compatibilité avec le PVC.
2) Préparation de plaques de PVC plastifié : Des plaques de PVC plastifié sont confectionnées à l'aide d'une presse de type CARVER et d'un moule de 30 x 30 cm en inox poli miroir muni d'un cadre de 2 mm d'épaisseur et d'un couvercle inox poli miroir. Une quantité de 180 g de poudre de PVC plastifié préparée à l'étape 1) est versée uniformément dans le cadre placé à l'intérieur du moule, puis le tout est recouvert d'un couvercle. L'ensemble est placé sur le plateau de la presse préchauffée à 185°C et le programme qui consiste en l'application d'une force de fermeture de 18 000 kg à 185°C pendant 2 min est lancé. Après refroidissement jusqu'à une température proche de 45°C, la plaque de PVC ainsi obtenue est alors démoulée.

Le tableau ci-dessous présente les valeurs de point sec obtenues lors de l'étape de mélange à sec avec les composés des exemples 1 à 6, ainsi que l'aspect de la plaque de PVC plastifié en sortie de presse.

| | Point sec (s) | Aspect de la plaque de PVC plastifié |
|---|---|---|
| Exemple 1 | 1474 - 1218 - 1380 | Non cohésive, cassante, opaque, grasse |
| Exemple 2 | 1343 - 1402 - 1261 | Non cohésive, cassante, opaque, grasse |
| Exemple 3 | 1352 - 1247 - 1499 | Non cohésive, cassante, opaque, grasse |
| Exemple 4 | 466 - 403 - 451 | Cohésive, transparente et souple |
| Exemple 5 | 442 - 516 - 464 | Cohésive, transparente et souple |
| Exemple 6 | 529 - 493 - 458 | Cohésive, transparente et souple |

Ce tableau montre que les composés des exemples 4 à 6 selon l'invention sont beaucoup plus compatibles avec le PVC que les composés des exemples 1 à 3 hors invention. De manière très avantageuse, les composés des exemples 4 à 6 possèdent une très bonne aptitude à plastifier le PVC en permettant la production de plaque de PVC flexible d'aspect souple et transparent, quand les exemples 1 à 3 hors invention ne le permettent pas.

## Revendications

1. Composé de formule (I) suivante :

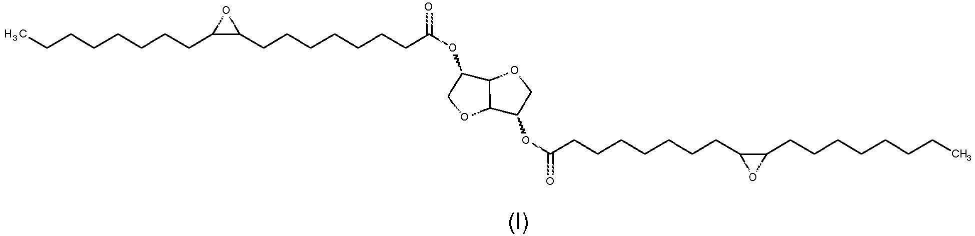

2. Composé de formule (II) suivante :

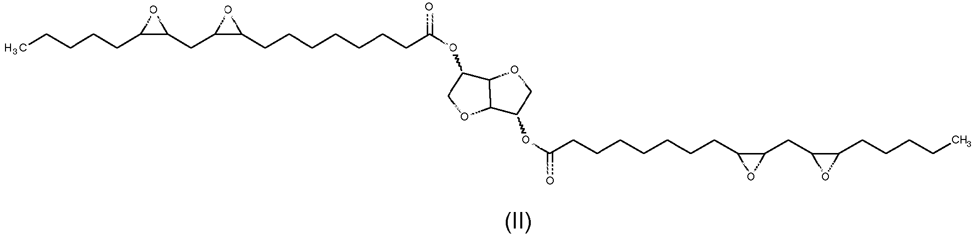

3. Composé de formule (III) suivante :

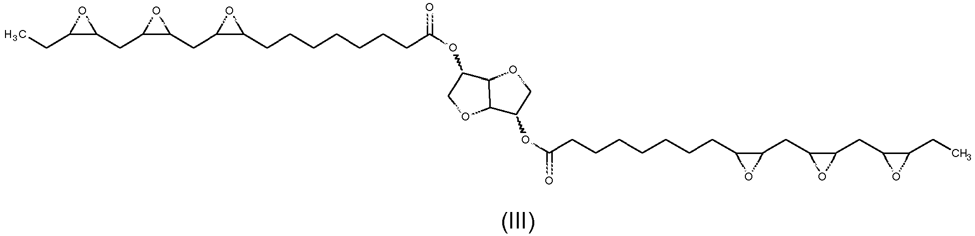

4. Mélanges de composés de formules (I), (II) et (III) :

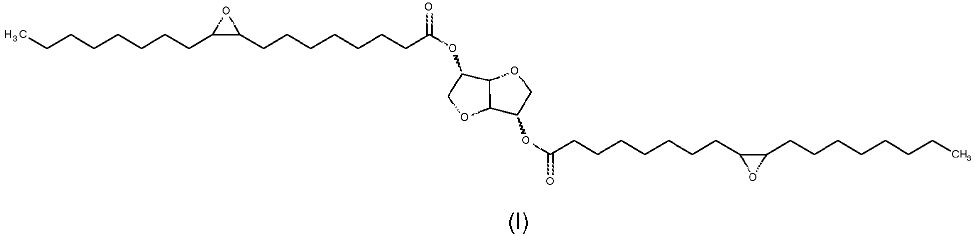

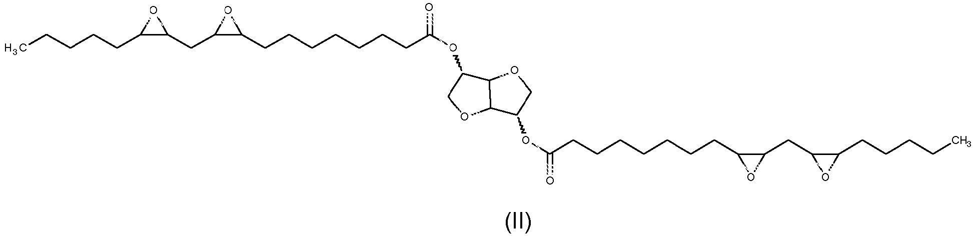

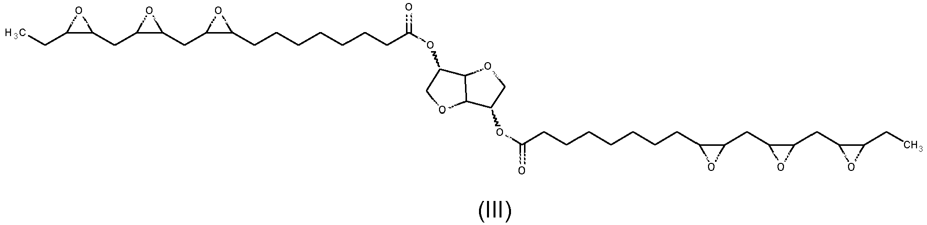

5. Formulation de polychlorure de vinyle comprenant du polychlorure de vinyle et l'un des composés ou mélange selon l'une quelconque des revendications 1 à 4.

6. Article plastique comprenant du polychlorure de vinyle et l'un des composés ou mélange selon l'une quelconque des revendications 1 à 4.

7. Utilisation d'un composé ou d'un mélange selon l'une quelconque des revendications 1 à 4 comme plastifiant dans une formulation de polychlorure de vinyle.

8. Procédé de préparation d'une formulation de polychlorure de vinyle comprenant le mélange d'un polychlorure de vinyle avec un composé ou un mélange selon l'une quelconque des revendications 1 à 4.

## Patentansprüche

1. Zusammensetzung mit der folgenden Formel (I):

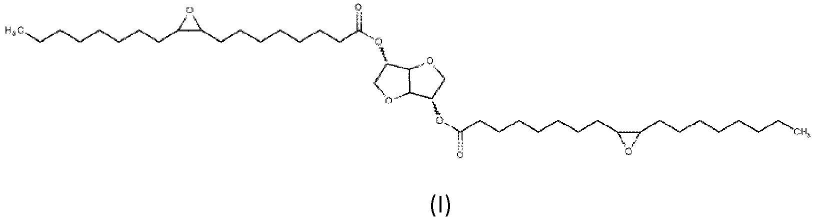

2. Zusammensetzung mit der folgenden Formel (II):

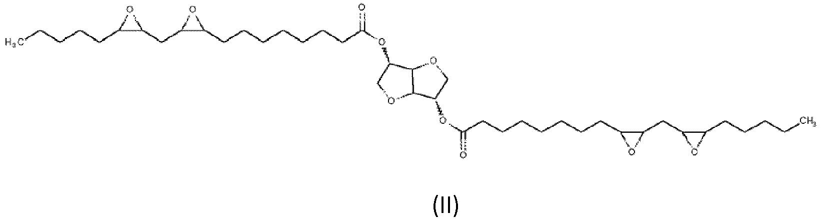

3. Zusammensetzung mit der folgenden Formel (III):

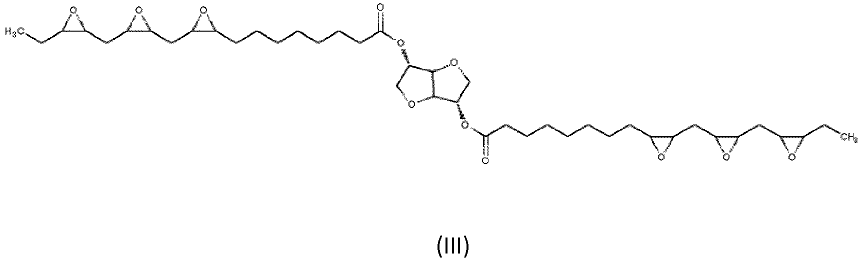

4. Mischungen von Zusammensetzungen mit den Formeln (I), (II), und (III):

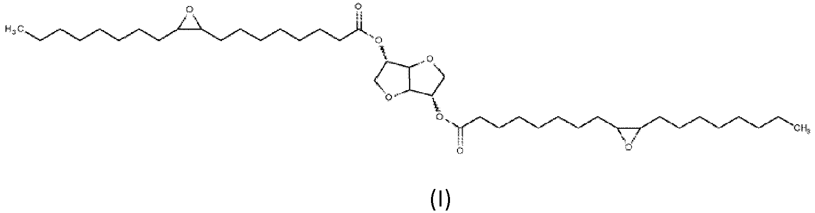

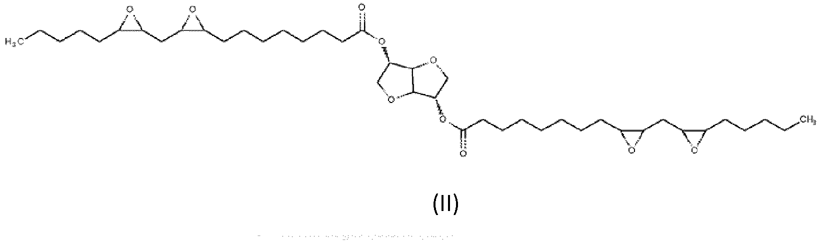

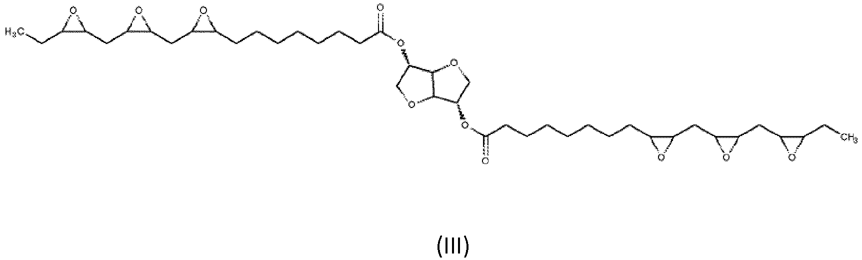

5. Polyvinylchlorid-Formulierung, welche Polyvinylchlorid und eine der Zusammensetzungen oder eine Mischung nach einem der Ansprüche 1 bis 4 umfasst.

6. Kunststoffartikel, welcher Polyvinylchlorid und eine der Zusammensetzungen oder eine Mischung nach einem der Ansprüche 1 bis 4 umfasst.

7. Verwendung einer Zusammensetzung oder einer Mischung nach einem der Ansprüche 1 bis 4 als Weichmacher in einer Polyvinylchlorid-Formulierung.

8. Verfahren der Herstellung einer Polyvinylchlorid-Formulierung, welche die Mischung eines Polyvinylchlorids mit einer Zusammensetzung oder einer Mischung nach einem der Ansprüche 1 bis 4 umfasst.

## Claims

1. Compound having the following formula (I):

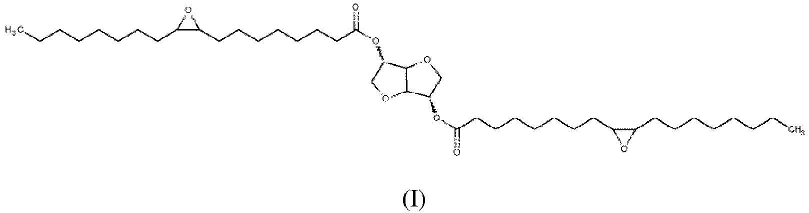

2. Compound having the following formula (II):

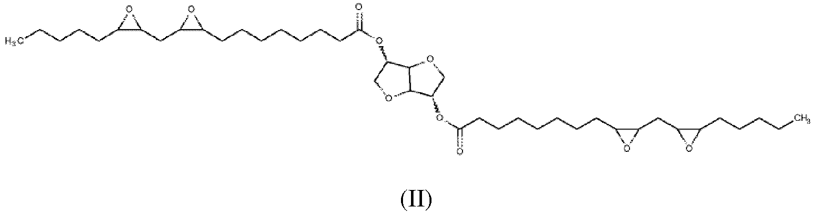

3. Compound having the following formula (III):

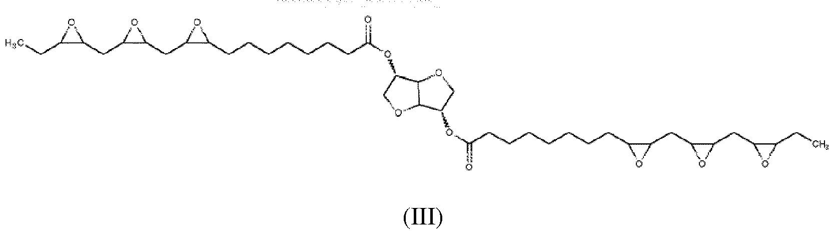

4. Mixtures of compounds having formulas (I), (II) and (III):

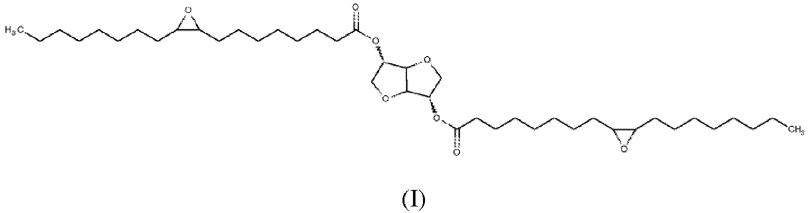

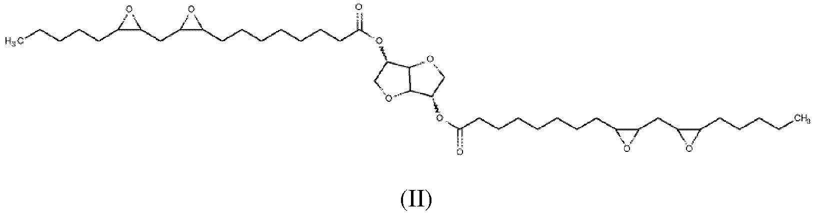

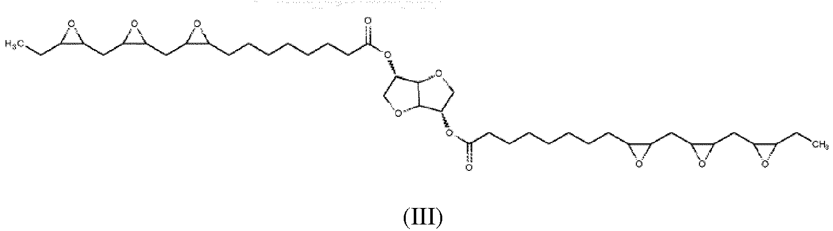

5. Formulation of polyvinyl chloride comprising polyvinyl chloride and one of the compounds or mixture according to any of claims 1 to 4.

6. Plastic article comprising polyvinyl chloride and one of the compounds or mixture according to any of claims 1 to 4.

7. Use of a compound or of a mixture according to any of claims 1 to 4 as a plasticizer in a formulation of polyvinyl chloride.

8. Method for preparing a formulation of polyvinyl chloride comprising the mixture of a polyvinyl chloride with a compound or a mixture thereof according to any of claims 1 to 4.
